# EUROPEAN PATENT APPLICATION

(11) **EP 2 412 802 A1**
(43) Date of publication of application: **01.02.2012**
(21) Application number: 10368030.2
(22) Date of filing: 29.07.2010
(51) Int. Cl.: C12N 5/0783

(54) **IL-13 producing TR1-like cells and use thereof**

(71) Applicant: TXCell, 06560 Valbonne (FR)
(72) Inventor: Foussat, Arnaud, 06410 Biot (FR); Bastian, Hervé, 06810 Auribeau-sur-Siagne (FR); Brun, Valérie, 06410 Biot (FR); Quatannens, Brigitte, 06210 Mandelieu-la-Napoule (FR)

(57) **Abstract**

The present invention relates to an isolated Tr1-like cell population capable of producing IL-13, said population having immunosuppressive activities; methods for identifying/isolating/enriching said population and its uses thereof.

## Description

### FIELD OF THE INVENTION

The present invention relates to isolated IL-13 producing Tr1-like cells, methods for identifying/isolating/enriching thereof and methods and kits for diagnosing or treating inflammatory diseases, autoimmune diseases, allergic diseases and organ transplantation conditions using isolated IL-13 producing Tr1-like cells.

### BACKGROUND OF THE INVENTION

Homeostasis of the immune system depends on the balance between immune response to an invading pathogen and immune tolerance to self-antigens. Both central and peripheral tolerances are important mechanisms for the induction and maintenance of T cell tolerance. In the last decade, much attention has been paid to regulatory T cells (Treg) that play a significant role in maintaining peripheral immune tolerance. It has now been firmly established that Treg can be divided into two different subtypes: natural Treg (nTreg) and inducible Treg populations such as TGF-beta induced Treg cells, Tr1 cells or Th3 cells.

nTreg are mainly described as FoxP3+ T cells whereas Tr1 cells are described as IL-10 producing type 1 cells (Allan et al, 2008, Immunological reviews, 223:391-421). FoxP3+ nTregs suppress/modulate a wide variety of different immune cells including naive and memory CD4+ and CD8+ T effector cells, B cells, monocytes and dendritic cells. Tr1 cells regulate immune responses by secreting IL-10 and transforming growth beta factor (TGF-β) and have the capacity to suppress both naive and memory T cell responses.

The inventors found a new type of regulatory T cells, which are Tr1-like and which are capable of producing IL-13.

### SUMMARY OF THE INVENTION

One object of the invention is an isolated Tr1-like cell population capable of producing IL-13. In one embodiment of the invention, said isolated Tr1-like cell population produce low amounts of IL-4. In another embodiment of the invention, said isolated Tr1-like cell population express:
- CD4 and
- a low level of CD127 and
- a low level CD62L.

In another embodiment of the invention, said isolated Tr1-like cell are resting cells and express a low level of CD25 and a low level of FoxP3. In another embodiment of the invention, said isolated Tr1-like cell population is activated and express CD25 and an intermediate level of FoxP3.

Another object of the invention is an isolated Tr1-like clone capable of producing IL-13. In one embodiment of the invention, said isolated Tr1-like clone produce low amounts of IL-4. In another embodiment, said Tr1-like clone expresses:
- CD4 and
- a low level of CD127 and
- a low level CD62L.

Another object of the invention is a method of identifying an IL-13 producing Tr1-like cell population, comprising:
- detecting the cell surface expression of CD4, and at least one of CD127 and CD62L markers on a cell population,
- detecting the production of IL-13,
wherein the cells expressing CD4 and a low level of CD127 and/or a low level

CD62L and producing IL-13, are the IL-13 producing Tr1-like cell population. Another object of the invention is a method for enriching a cell population in IL-13 producing Tr1-like cells, comprising:
- identifying the cells expressing CD4 and a low level of CD127 and/or a low level CD62L and producing IL-13,
- selecting said cells,
   thereby obtaining a cell population enriched in IL-13 producing Tr1-like cells wherein the percentage of IL-13 producing Tr1-like cells is at least twice the percentage of IL-13 producing Tr1-like cells before enrichment.

Another object of the invention is an enriched IL-13 producing Tr1-like cell population obtained according to the method here above described.

Another object of the invention is a kit for identifying or isolating an IL-13 producing Tr1-like cell population, comprising means for detecting the cell surface expression of CD4, CD25, CD127 and CD62L and means for detecting IL-13 production.

Another object of the invention is an isolated and/or enriched IL-13 producing Tr1-like cell population or the IL-13 producing Tr1-like clone for preventing or treating an immune response, graft versus host disease and organ rejection, allergic disease, an inflammatory condition or an autoimmune condition.

Another object of the invention is a method for depleting a cell population in IL-13 producing Tr1-like cells, comprising:
- identifying the IL-13 producing Tr1-like cells according to claim 9,
- depleting said cells,
thereby obtaining a cell population depleted in IL-13 producing Tr1-like cells, wherein the percentage of IL-13 producing Tr1-like cells is at least 0.5 fold the percentage of IL-13 producing Tr1-like cells before depletion.

Another object of the invention is an IL-13 producing Tr1-like cells-depleted cell population obtained according to the method as described here above.

Another object of the invention is an IL-13 producing Tr1-like cells-depleted cell population for increasing an immune response in a subject in need thereof.

### DETAILED DESCRIPTION OF THE INVENTION

The invention provides a new isolated IL-13 producing Tr1-like cell population having immunosuppressive capacities and uses thereof. The inventors found that these cells are Tr1-like cells as they express the same surface markers than Tr1 cells and that these cells are capable of producing IL-13 and low level of IL-4. The inventors found that their immunosuppressive capacities are mediated by IL-13.

One object of the invention is an isolated Tr1-like cell population capable of producing IL-13.

As used herein, the term "IL-13" refers to the interleukin 13.

As used herein, the term "producing IL-13" refers to the secretion of amount of IL-13 of more than 100 pg/ml in the culture medium, preferably more than 200 pg/ml, more preferably more than 500 pg/ml. In another way, it refers to the secretion of amount of IL-13 of more than 250pg/10⁶ IL-13-producing Tr1-like cells, preferably more than 500 pg/10⁶ IL-13-producing Tr1-like cells and more preferably more than 1000 pg/10⁶ IL-13-producing Tr1-like cells.

In one embodiment of the invention, said isolated Tr1-like cell population produce low amounts of IL-4

As used herein, the term "IL-4" refers to the interleukin 4.

As used herein, the term "low amounts of IL-4" refers to the amounts of less than 500 pg/ml, preferably less than 250 pg/ml, more preferably less than 100 pg/ml.

In another way, it refers to amounts of IL-4 of less than 500 pg/10⁶ IL-13-producing Tr1-like cells, preferably less than 250 pg/10⁶ IL-13-producing Tr1-like cells, more preferably less than 100 pg/10⁶ IL-13-producing Tr1-like cells.

Methods for evaluating if a cell is capable of producing IL-13 and producing low amounts of IL-4 are well known in the art. One example of these methods is the following: the cells are activated in the presence of anti-CD3 (10 microg./ml) and anti-CD28 (1 microg./ml) during 48h and cytokines secretion is measured at this point by ELISA or by FACS.

In another embodiment of the invention, said isolated Tr1-like cells express at their surface CD4 and a low level of CD127 and a low level of CD62L.

In one embodiment, said isolated Tr1-like cells are resting and do not express at their surface CD25.

In another embodiment, said isolated Tr1-like cells are activated and express at their surface CD25.

As used herein, "isolated" refers to a cell or a cell population that is removed from its natural environment (such as the peripheral blood) and that is isolated, purified or separated, and is at least about 75% free, 80% free, 85% free and preferably about 90%, 95%, 96%, 97%, 98%, 99% free, from other cells with which it is naturally present, but which lack the cell surface markers based on which the cells were isolated.

As used herein, the term "cell surface marker" refers to proteins, carbohydrates, or lipids on the surface of the cells that can be used to discriminate a cell population.

As used herein, the term "expression" refers interchangeably to expression of a gene or gene product, including the encoded polypeptide or protein. Expression of a gene product may be determined, for example, by immunoassay using an antibody(ies) that bind with the polypeptide. Alternatively, expression of a gene may be determined, by measurement of mRNA levels, for example, by RT-PCR, RT-qPCR.

The term "naive" is well known in the art and refers to an immune cell or a population of cells that have not yet encountered any specific antigen.

The term "resting" is well known in the art and refers to an immune cell or a population of cells that do not proliferate, do not produce cytokines and that do not express conventional immune cell activation molecules at the surface such as CD25.

The term "activated" is well known in the art and refers to an immune cell or a population of cells that proliferates and/or produces cytokines and expresses conventional immune cell activation molecules at its surface such as CD25.

Especially for T lymphocytes, the passage from a resting to an activated status is mediated by the encounter of the T cell with its specific antigen or by activation with activating cytokines or mitogens.

The term "low" or "lo" or "lo/-" as used in relation to CD127^{lo/-}, CD62L^{lo/-} or CD25L^{lo/-} is well known in the art and refers to the expression level of the cell marker of interest, in that the expression level of the cell marker is low by comparison with the expression level of that cell marker in the population of cells being analyzed as a whole. More particularly, the term "lo" refers to a distinct population of cells that express the cell marker at a lower level than one or more other distinct population of cells.

The term "high" or "hi" or "bright" is well known in the art and refers to the expression level of the cell marker of interest, in that the expression level of the cell marker is high by comparison with the expression level of that cell marker in the population of cells being analyzed as a whole.

The terms "+" and "-" are well known in the art and refer to the expression level of the cell marker of interest, in that the expression level of the cell marker corresponding to "+" is high or intermediate and the expression level of the cell marker corresponding to "-" is low or null. Generally, cells in the top 2, 3, 4, or 5% of staining intensity are designated "hi", with those falling in the top half of the population categorized as being "+". Those cells falling below 50%, of fluorescence intensity are designated as "lo" cells and below 5% as "-" cells.

As used herein, the term "CD127" refers to the "interleukin-7 receptor" (IL-7R) present on a cell surface. The IL-7 receptor alpha chain is described in the literature (e.g., Goodwin et al. (1990) Cell 60:941-951). IL-7R is also referred to in the literature as CD127. CD127⁺ refers to cells which stain intermediate or brightly when treated with a labeled antibody directed toward CD127. CD127^{lo/-} refers to cells of a type that stains slightly/dully or not at all when contacted with a labeled CD127 antibody. Generally, the cells are distinguished according to their CD127 expression levels based upon a readily discernible difference in staining intensity as it is known to one of ordinary skill in the art. In some embodiments, the cut off for designating a cell as a CD127^{lo/-} cell can be set in terms of the fluorescent intensity distribution observed for all the cells with those cells falling below the 50%, 40%, 30% or 20% of fluorescence intensity being designated as CD127^{lo/-} cells. A CD127⁻ cell can be designated as one that falls below the tenth bottom percentile with respect to fluorescence intensity. In some embodiments, the frequency distribution of the CD127 staining is obtained for all the cells and the population curve fit to a higher staining and lower staining population, and cells assigned to the population to which they most statistically are likely to belong in view of a statistical analysis of the respective population distributions. In some embodiments, the CD127^{lo/-} cells stain two to three folds less intensely than the CD127⁺ cells.

As used herein, the term "CD62L" refers to L-selectin which is a critical adhesion molecule for lymphocyte migration. CD62L⁺ refers to cells which stain intermediate or brightly when treated with a labeled antibody directed toward CD62L. CD62L^{lo/-} refers to cells of a type which stains slightly/dully or not at all when contacted with a labeled CD62L antibody. Generally, the cells are distinguished according to their CD62L expression levels based upon a readily discernible difference in staining intensity as it is known to one of ordinary skill in the art. In some embodiments, the cut off for designating a cell as a CD62L^{lo/-} cell can be set in terms of the fluorescent intensity distribution observed for all the cells with those cells falling below the 50%, 40%, 30% or 20% of fluorescence intensity being designated as CD62L^{lo/-} cells. A CD62L⁻ cell can be designated as one that falls below the tenth bottom percentile with respect to fluorescence intensity. In some embodiments, the frequency distribution of the CD62L staining is obtained for all the cells and the population curve fit to a higher staining and lower staining population, and cells assigned to the population to which they most statistically are likely to belong in view of a statistical analysis of the respective population distributions. In some embodiments, the CD62L^{lo/-} cells stain two to three fold less intensely than the CD62L⁺ cells.

As used herein, the term "CD4" refers to a cell-surface glycoprotein typically found on the mature helper T cells and immature thymocytes, as well as on monocytes and macrophages. On T cells, CD4 is the co-receptor for the T cell receptor (TCR) and recruits the tyrosine kinase lck. With its D1-portion, CD4 can attach to the beta2-domain of MHC class II molecules. CD4⁺ refers to cells which stain brightly when contacted with labeled anti-CD4 antibody, and CD4⁻ refers to cells of a type which stain the least brightly, dull or not at all, when contacted with a fluorescently labeled CD4 antibody. Generally, the cells are distinguished according to their CD4 expression levels based upon a readily discernible difference in staining intensity as the CD4 staining is clearly bimodal. In some embodiments, the frequency distribution of the CD4 staining is obtained for all the cells and the population curve fit to a higher staining and lower staining population, and cells assigned to the population to which they most statistically are likely to belong in view of a statistical analysis of the respective population distributions. In some embodiments, the CD4⁻ cells stain two to three fold less intensely than the CD4⁺ cells.

As used herein, the term "CD25" refers to the alpha subunit of interleukin-2 receptor, a single-chain glycoprotein with a molecular weight of 55 kD. Following the activation of T cells with antigen or mitogen in the presence of the monokine interleukin-1, interleukin 2 (IL-2) is rapidly synthesized and secreted. In response to this, a subpopulation of T cells expresses high affinity receptors for IL-2. These cells proliferate, expanding the T cell population which is capable of mediating helper, suppressor and cytotoxic functions. IL-2 receptor is not uniquely found on T cells. CD25^{hi} refers to cells which stain brightly when contacted with labeled anti-CD25 antibody, CD25⁺ refers to cells which stain less brightly when contacted with labeled anti-CD25 antibody, and CD25^{lo/-} refers to cells which are of a type which stains the least brightly dull or null when contacted with a labeled CD25 antibody. Generally, the cells are distinguished according to their CD25 expression levels based upon differences in staining intensity as is known to one of ordinary skill in the art. In some embodiments, the cut off for designating a cell as a CD25 expression category hi, +, lo, or - cell can be set in terms of the fluorescent intensity distribution observed for all the cells. Generally, cells in the top 2, 3, 4, or 5% of staining intensity are designated "hi", with those falling in the top half of the population categorized as being "+". Those cells falling below 50%, of fluorescence intensity are designated as CD25^{lo} cells and below 5% as CD25⁻ cells.

Said Tr1-like cells can thus be operationally characterized by their cell surface markers and their capacity to produce IL-13. These cell surface markers can be recognized by reagents that specifically bind to the cell surface markers. For example, proteins, carbohydrates, or lipids on the surface of Tr1 cells can be immunologically recognized by antibodies specific for the particular protein or carbohydrate (for use of antibodies to markers, see, Harlow, Using Antibodies: A Laboratory Manual (Cold Spring Harbor Press, Cold Spring Harbor, N.Y., 1999); see also, EXAMPLES). The set of markers present on the surface of Tr1-like cells and absent from the surface of these cells is characteristic for Tr1-like cells. Therefore, Tr1-like cells can be selected by positive and negative selection using cell surface markers. A reagent that binds to a cell surface marker expressed by a Tr1-like cell, a "positive marker", can be used for the positive selection of Tr1-like cells (i.e., retaining cells that express the cell surface marker). Conversely, negative selection relies on that fact that certain cell surface markers are not expressed by Tr1-like cells. Therefore, a "negative marker" (i.e., a marker not present on the cell surface of Tr1-like cells) can be used for the elimination of those cells in the population that are not Tr1-like cells by the removal of cells that bind to the reagent specific for the negative marker.

In one embodiment, discrimination between cells based upon the detected expression of cell surface markers occurs by comparing the expression of a cell surface marker with the mean expression by a control population of cells. For example, the expression of a marker on a Tr1-like cell can be compared to the mean expression of the same marker on other cells derived from the same sample as the Tr1-like cell. Other methods of discriminating among cells by marker expression include gating Cells by flow cytometry using a combination of reagents (see, Givan A, Flow Cytometry: First Principles, (Wiley-Liss, New York, 1992); Owens M A & Loken M R., Flow Cytometry: Principles for Clinical Laboratory Practice, (Wiley-Liss, New York, 1995)).

By a "combination of reagents" is meant at least two reagents that bind to cell surface markers either present (positive marker) or not present (negative marker) on the surfaces of Tr1-like cells, or that bind to a combination of positive and negative markers. For example, the use of a combination of antibodies specific for Tr1-like cell surface markers results in isolation and/or enrichment of Tr1-like cells from a variety of samples/tissues.

An "anti-X antibody" or "X antibody" according to the invention is an antibody which can specifically bind to X. For instance, the anti-CD127 antibody or CD127 antibody is capable of binding CD127. The antibodies for use according to the invention include, but are not limited to, recombinant antibodies, polyclonal antibodies, monoclonal antibodies, chimeric antibodies, human monoclonal antibodies, humanized or primatized monoclonal antibodies, and antibody fragments. A great many lymphocyte biomarker specific antibodies are commercially available. These include anti-CD127, anti-CD4, anti-CD62L, anti-CD25, anti-IL-4, anti-IL-10 and anti-IL-13 antibodies (R&D, BD Biosciences....). "Antibody" refers to a polypeptide comprising a framework region from an immunoglobulin gene or fragments thereof that specifically binds and recognizes an antigen. The recognized immunoglobulin genes include the kappa, lambda, alpha, gamma, delta, epsilon, and mu constant region genes, as well as the myriad immunoglobulin variable region genes. Light chains are classified as either kappa or lambda. Heavy chains are classified as gamma, mu, alpha, delta, or epsilon, which in turn define the immunoglobulin classes, IgG, IgM, IgA, IgD and IgE, respectively. Typically, the antigen-binding region of an antibody will be most critical in specificity and affinity of binding. An exemplary immunoglobulin (antibody) structural unit comprises a tetramer. Each tetramer is composed of two identical pairs of polypeptide chains, each pair having one "light" (about 25 kD) and one "heavy" chain (about 50-70 kD). The N-terminus of each chain defines a variable region of about 100 to 110 or more amino acids primarily responsible for antigen recognition. The terms variable light chain (VL) and variable heavy chain (VH) refer to these light and heavy chains respectively. Antibodies exist, e.g., as intact immunoglobulins or as a number of well-characterized fragments produced by digestion with various peptidases. Thus, for example, pepsin digests an antibody below the disulfide linkages in the hinge region to produce F(ab)'2, a dimer of Fab which itself is a light chain joined to VH-CH1 by a disulfide bond. The F(ab)'2 may be reduced under mild conditions to break the disulfide linkage in the hinge region, thereby converting the F(ab)'2 dimer into an Fab' monomer. The Fab' monomer is essentially Fab with part of the hinge region (see Fundamental Immunology (Paul ed., 3d ed. 1993)). While various antibody fragments are defined in terms of the digestion of an intact antibody, one of skill will appreciate that such fragments may be synthesized de novo either chemically or by using recombinant DNA methodology. Thus, the term antibody, as used herein, also includes antibody fragments either produced by the modification of whole antibodies, or those synthesized de novo using recombinant DNA methodologies (e.g., single chain Fv) or those identified using phage display libraries (see, e.g., McCafferty et al., Nature 348:552-554 (1990)). In some embodiments, a high affinity ligand of a target may be used in place of the antibody. The phrase "specifically (or selectively) binds" to an antibody or "specifically (or selectively) immunoreactive with," when referring to a protein or peptide, refers to a binding reaction that is determinative of the presence of the protein, often in a heterogeneous population of proteins and other biologics. Specific binding to an antibody under such conditions requires an antibody that is selected for its specificity for a particular protein. For example, polyclonal antibodies can be selected to obtain only those polyclonal antibodies that are specifically immunoreactive with the selected antigen and not with other proteins. This selection may be achieved by subtracting out antibodies that cross-react with other molecules.

Preferably a "label" or a "detectable moiety" is covalently or non covalently attached to the antibody. A label may be detectable by spectroscopic, photochemical, biochemical, immunochemical, chemical, or other physical means. Particularly useful labels are fluorescent dyes. Methods of attaching labels to antibodies are well known to those of ordinary skill in the art. Particularly preferred labels are those which are attached to the antibody by a linker which can be readily cleaved or separated or subject to hydrolysis by contact with a predetermined enzyme under physiological conditions. The antibody may also be conjugated with a magnetic particle, such as a paramagnetic microbead (Miltenyi Biotec, Germany). An activated T cell bound by a magnetically labeled antibody may be isolated using techniques including, but not limited to, magnetic cell sorting. Suitably labeled antibodies to CD127, CD62L, CD4 and CD25, as well as many other cluster of differentiation, are commercially available and known to one of ordinary skill in the art. The antibody may be labeled before or after contact with the sample or before or after contact with the CD. The CD antibody may be labeled by contacting with a labeled antibody which binds to the CD-antibody. As used herein, the term "CD" or "cluster of differentiation" or "common determinant" refers to cell surface molecules recognized by antibodies.

In one embodiment of the invention, the IL-13 producing Tr1-like cell population is resting and the cells express a low level of FoxP3.

In another embodiment of the invention, the IL-13 producing Tr1-like cell population is activated and the cells express an intermediate level of FoxP3.

As used herein, the term "Foxp3" refers to the nuclear protein Foxp3 believed to act as a transcription factor (Hori et al., 2003; Yasayko, J. E. et al., Nat. Genet. 27:68-73 (2001); Fontenot, J. D. et al., Nat. Immunol. 4:330-336 (2003); Khattri, R. et al., Nat. Immunol. 4:337-342 (2003)). As being intra cellular located, Foxp3 expression may be assessed by intracellular flow cytometry using a labeled anti-Foxp3 antibody (eBioscience inc or BD biosciences).

Another object of the invention is a composition comprising, consisting essentially of or consisting of the isolated IL-13 producing Tr1-like cells.

Another object of the invention is a method for identifying/isolating/quantifying an IL-13 producing Tr1-like cell population, comprising:
- detecting the cell surface expression of CD4, and one of CD127 and CD62L on a cell population,
- detecting the production of IL-13,
wherein the cells expressing CD4 (i.e. being CD4⁺) and a low level of CD127 and/or a low level of CD62L (i.e. being CD127^{lo/-} and CD62L^{lo/-}) and that producing IL-13 are the IL-13 producing Tr1-like cells, and
- identifying/isolating/quantifying said cells.

As mentioned here above, the cell surface expression of CD4, CD127 and CD62L may be detected by using antibodies anti-CD4, -CD62L and -CD127.

The detection of the production of IL-13 may be carried out by different means well known in the art.

For example, a first method for detecting IL-13 production relies on the use of a labeled antibody anti-IL-13 for an intracellular staining of T cells previously permeabilized, the staining being read by FACS.

Another example of a method for detecting IL-13 production relies on an ELISA test carried out with a labeled anti-IL13.

Finally, another example of a method for detecting IL-13 production relies on the use of the IL-13 secretion assay kit commercialized by Miltenyi Biotech (130-093-480) or an equivalent thereof. This method is particularly of interest as it allows the detection and the enrichment of cells producing IL-13 without having to permeabilize them.

Any cellular source that contains T cells can be used to isolate IL-13 producing Tr1-like cells. Useful sources include, but are not limited to, peripheral blood, synovial fluid, spleen, thymus, lymph nodes, bone marrow, Peyer's patches, and tonsils.

Methods for separating, isolating, or selecting cells include, but are not limited to magnetic separation using antibody-coated magnetic beads (Schwartz, et al, U.S. Pat. No. 5,759,793) and affinity chromatography or "panning" using antibody attached to a solid matrix (e.g. a plate). Further techniques providing accurate separation include fluorescence-activated cell sorters (FACS), which can have varying degrees of sophistication, such as having multiple color channels, low angle and obtuse light scattering detecting channels, or impedance channels. Dead cells can be eliminated by selection with dyes associated with dead cells e.g., (propidium iodide, LDS). Red blood cells can be removed by, for example, elutriation, hemolysis, or Ficoll-Paque gradients. Any technique can be employed that is not unduly detrimental to the viability of the selected cells.

Conveniently, antibodies can be conjugated with labels for a number of different purposes: e.g., magnetic beads to allow for ease of separation of Tr1 cells; biotin, which binds with high affinity to avidin or streptavidin; fluorochromes, which can be used with a fluorescence activated cell sorter; haptens; and the like. Multi-color analyses can be employed with FACS or in a combination of immunomagnetic separation and flow cytometry. Multi-color analysis is of interest for the separation of cells based on multiple surface antigens: e.g., non-CD4⁺ immune cell markers (CD8, CD14, CD16, CD19, CD36, CD56, CD123, TCRgamma/delta and glycophorin A), CD4, CD25, CD127 and CD62L. Fluorochromes which find use in a multi-color analysis include, but are not limited to, phycobiliproteins, e.g. phycoerythrin and allophycocyanins; fluorescein, and Texas red. Magnetic separation is a process used to selectively retain magnetic materials within a vessel, such as a centrifuge tube or column, in a magnetic field gradient. Tr1 cells can be magnetically labeled by binding magnetic particles to the surface of the cells through specific interactions, including immuno-affinity interactions. The suspension, containing the Tr1 cells within a suitable vessel, is then exposed to magnetic field gradients of sufficient strength to separate the Tr1 cells from other cells in the suspension. The vessel can then be washed with a suitable fluid to remove the unlabeled cells, resulting in a purified suspension of Tr1 cells.

The majority of magnetic labeling systems use superparamagnetic particles with monoclonal antibodies or streptavidin covalently bound to their surface. In cell separation applications, these particles can be used for either positive selection, where the cells of interest are magnetically labeled and retained, or negative selection where the majority of undesired cells are magnetically labeled and retained. The diameter of the particle used varies widely from about 50-100 nm for MACS particles (Miltenyi Biotec) and StemSep(TM) colloid (Stem Cell Technologies), through 150-450 nm for EasySep(R) (Stem Cell Technologies) and Imag particles (BD Biosciences), up to 4.2 µm for Dynabeads (Dynal Biotech). The type of particle used is influenced by the magnet technology employed to separate the labeled cells.

There are two important classes of magnetic separation technologies, both of which, for convenience and for practical reasons, use permanent magnets as opposed to electromagnets. The first class is column-based high-gradient-magnetic-field separation technology that uses small, weakly magnetic particles to label the targets of interest, and separates these targets in a column filled with a magnetizable matrix. Very high gradients are generated close to the surface of the matrix elements when a magnetic field is applied to the column. The high gradients are necessary to separate targets labeled with these relatively weakly magnetic particles. The second class is tube-based technology that uses more strongly magnetic particles to label the targets of interest. These targets of interest are then separated within a centrifuge-type tube by magnetic field gradients generated by a magnet outside the tube. This method has the advantage that it does not rely on a magnetizable matrix to generate the gradients; and, therefore does not require an expensive disposable column or a reusable column with an inconvenient cleaning and decontamination procedure.

Once placed within the magnet, targeted cells migrate toward the region or regions of highest magnetic field strength and are retained within the magnetic field while the unlabeled cells are drawn off. The targeted cells can then be collected and used after removal from the magnetic field. In the event that negative selection is required, the unlabeled cells are retained and can be utilized for a variety of applications.

FACS permits the separation of sub-populations of cells on the basis of their light scatter properties as they pass through a laser beam. The forward light scatter (FALS) is related to cell size, and the right angle light scatter, also known as side scatter characteristic (SSC) is related to cell density, cellular content and nucleo-cytoplasmic ratio, i.e. cell complexity. Since cells can be labeled with fluorescent-conjugated antibodies, they can further be characterized by antibody (fluorescence) intensity.

In particular embodiments, the IL-13 producing Tr1-like cells of the present invention, are isolated using immuno-magnetic chromatography. For example, an anti-CD4 antibody is attached to magnetic beads. These antibody-labeled magnetic beads are used as the basis for the affinity purification. The antibody-labeled fraction of T cells is applied to the magnetic affinity column. The non-adherent cells are discarded and the adherent cells are eluted from the magnetic column by removal of the magnetic field. In another embodiment, the cells are first labeled with an antibody (e.g., anti-CD4) and then labeled with a secondary antibody carrying a magnetic bead or sphere.

In another embodiment, a secondary antibody immunoreactive with a Tr1 cell can be used to enrich the population of IL-13 producing Tr1-like cells. The use of a secondary antibody is generally known in the art. Typically, secondary antibodies are antibodies immunoreactive with the constant regions of the first antibody. Preferred secondary antibodies include anti-rabbit, anti-mouse, anti-rat, anti-goat, and anti-horse immunoglobulins and are available commercially. Commercially available kits provide secondary antibodies conjugated to labeling agents such as, but limited to, magnetic particles and fluorochromes.

Another object of the invention is a method for enriching a cell population in IL-13 producing Tr1-like cells, comprising:
- identifying the cells expressing CD4 and a low level of CD127 and/or CD62L and producing IL-13,
- selecting said cells,
thereby obtaining a cell population enriched in IL-13 producing Tr1-like cells.

In another embodiment of the invention, a method for enriching a population in IL-13 producing Tr1-like cells may comprise:
- contacting the cell population with at least one reagents which binds to a marker of non-CD4⁺ cells, thereby depleting non-CD4⁺ cells,
- identifying IL-13 producing Tr1-like cells as described here above and selecting them.

Examples of markers that bind to non-CD4⁺ cells include, but are not limited to, CD8, CD14, CD16, CD19, CD36, CD56, CD123, and glycophorin A.

Preferred reagents that bind to said markers are antibodies. In a further embodiment, the antibodies are conjugated to a fluorochrome or magnetic particle. In another embodiment, the cell selection is performed by flow cytometry, fluorescence activated cell sorting, magnetic selection, affinity chromatography or panning or combinations thereof.

Another object of the invention is an enriched population of IL-13 producing Tr1-like cells obtained according to said methods.

Another object of the invention is a composition comprising, consisting essentially of or consisting of the enriched IL-13 producing Tr1-like cell population.

As used herein, an IL-13 producing Tr1-like cells enriched composition/population is one in which the percentage of IL-13 producing Tr1-like cells is higher than the percentage of IL-13 producing Tr1-like cells in the originally obtained population of cells. Although possible, an enriched population of IL-13 producing Tr1-like cells need not contain a homogenous population of IL-13 producing Tr1-like cells. In particular embodiments, at least about 50%, about 55%, about 60%, about 65%, about 70%, about 75%, about 80%, about 85%, about 90%, about 95%, about 98%, or about 99% of said cells of the composition are IL-13 producing Tr1-like cells. In another embodiments, the percentage of IL-13 producing Tr1-like cells in the enriched composition/population is at least twice, 5 times, 10 times, 20 times, 30 times, 40 times, 50 times, 60 times, 70 times, 80 times, 90 times, 100 times the percentage of IL-13 producing Tr1-like cells before enrichment.

Any cellular source that contains T cells can be used to isolate/enrich IL-13 producing Tr1-like cells. Useful sources include, but are not limited to, peripheral blood, synovial fluid, spleen, thymus, lymph nodes, bone marrow, Peyer's patches, and tonsils. Enrichment methods are variable based on the level of enrichment associated with each step of the enrichment process. The level of enrichment and percent purity of the IL-13 producing Tr1-like cells will depend on many factors including, but not limited to, the donor, the cell/tissue source and the disease state of the donor.

Another object of the invention is an IL-13 producing Tr1-like clone.

According to the invention, IL-13 producing Tr1-like cells are isolated according to the method here above described and then an IL-13 producing Tr1-like cell is cloned by methods well known in the art.

In another embodiment, the person skilled in the art can isolate the cells expressing CD4 and a low level of CD127 and/or CD62L, clone said cells and then isolate the clones producing IL-13 when activated.

Another object of the invention is a composition comprising, consisting essentially of or consisting of the IL-13 producing Tr1-like clone.

In one embodiment of the invention, the IL-13 producing Tr1-like clone produce low levels of IL-4.

The IL-13 producing Tr1-like cells isolated by the method of the invention may be further expanded by the in vitro method described in WO2006/108882. Said method comprises:
a) cultivating at a temperature T1 inferior to 35°C, in a culture medium Mf, feeder cells such as insect feeder cells, said temperature T1 allowing the proliferation of feeder cells and said feeder cells expressing factors which interact with the following cell surface proteins:
   - the CD3/TCR complex,
   - the CD28 protein,
   - the IL-2 receptor,
   - the CD2 protein,
   - the IL-4 receptor,
b) contacting the feeder cells obtained in step a) cleared or not of their culture medium Mf, with the IL-13 producing Tr1-like cell population contained in the culture medium Mp, wherein said culture medium Mp does not initially contain the factors cited in step a), in order to obtain a mixture containing the Tr1 cell population, the feeder cells and the culture medium Mp,
c) cultivating the mixture obtained at step b) at a temperature T2 which is at least 35°C, said temperature being chosen such that the Tr1 cell population proliferates and the feeder cells do not proliferate,
d) recovering the Tr1 cell population such expanded.

Examples of factors which interact with the above mentioned cell surface proteins include:
- a modified anti-CD3 antibody, wherein the anti-CD3 intracytoplasmic domain of the CD3 heavy chain is replaced with a transmembrane domain,
- the CD80 or CD86 protein,
- the IL-2 secreted by the feeder cells,
- the CD58 protein,
- an interleukin selected from the group comprising IL-4 and IL-13.

Mp medium is adapted to the culture of T cells and may be a serum-free culture medium. Non-limiting examples of Mp medium are commercially available serum-free medium such as XVIVO 15 from BioWhittaker, AIM V medium from Invitrogen, DMEM, RPMI....) Mf medium is adapted to the culture of feeder cells and may be serum-free culture medium. Non-limiting examples of Mf medium are commercially available serum-free medium such as Schneider's medium without serum from BioWhittaker, MD Gibco serum-free insect cell culture medium as SFM from Invitrogen or Insectagro from Krackeler Scientific Inc).

In an embodiment of the invention, the Tr1 cells thus obtained may be cloned by using conventional methods for cloning T cells.

In an embodiment of the present invention, the Tr1 cells thus obtained or the Tr1 cell clones may be frozen to be stored.

Another object of the invention is a composition comprising, consisting essentially of or consisting of the enriched and expanded IL-13 producing Tr1-like cell population.

Another object of the invention is a method for depleting a cell population in IL-13 producing Tr1-like cells, comprising:
- identifying the cells expressing CD4 and a low level of CD127 and/or CD62L and producing IL-13,
- depleting said cells,
- thereby obtaining a cell population depleted in IL-13 producing Tr1-like cells.

Another object of the invention is a composition comprising, consisting essentially of or consisting of the IL-13 producing Tr1-like cells-depleted population.

An IL-13 producing Tr1-like cell-depleted composition/population is one in which the percentage of IL-13 producing Tr1-like cells is lower than the percentage of IL-13 producing Tr1-like cells in the originally obtained population of cells.

Another object of the invention is an IL-13 producing Tr1-like cells-depleted cell population obtained according to the method as described here above.

As explained here above, methods for isolating cells include, but are not limited to, magnetic separation using antibody-coated magnetic beads, affinity chromatography or "panning" using antibody attached to a solid matrix (e.g. a plate) and fluorescence-activated cell sorters (FACS), Instead of being selected, the Tr1 cells are depleted.

In one embodiments, the percentage of IL-13 producing Tr1-like cells in the depleted composition/population is at least 0.5 times, 0.4 times, 0.3 times, 0.25 times, 0.2 times, 0.15 times, 0.1 times the percentage of IL-13 producing Tr1-like cells before depletion.

Another object of the invention is a kit for identifying or isolating an IL-13 producing Tr1-like cell population, or for enriching or depleting a cell population in IL-13 producing Tr1-like cells, said kit comprising reagents for detecting the cell surface expression of CD4, CD25, CD127 and CD62L and means for detecting IL-13 production. Preferably, said reagents are antibodies. More preferably, these antibodies are conjugated to a fluorochrome or magnetic particle.

In another embodiment, said kit further comprises a reagent for detecting Foxp3 expression. Preferably, said reagent is an antibody. More preferably, said antibody is conjugated to a fluorochrome.

Another object of the present invention is a method for inhibiting an immune response in a subject in need thereof, comprising the administration of an effective amount of the isolated or enriched IL-13 producing Tr1-like cells according to the invention to the subject.

In one embodiment, the subject has undergone or is undergoing transplantation such as bone marrow transplantation or organ transplantation.

In another embodiment, the subject has an allergy.

Another object of the present invention is a method for preventing or treating an inflammatory condition in a subject in need thereof, comprising the administration of an effective amount of the isolated or enriched IL-13 producing Tr1-like cells according to the invention to the subject.

Another object of the present invention is a method for preventing or treating an autoimmune condition in a subject in need thereof, comprising the administration of an effective amount of the isolated or enriched IL-13 producing Tr1-like cells according to the invention to the subject.

In one embodiment of the invention, the population of cells may be obtained from the subject into whom the IL-13 producing Tr1-like cells-enriched composition is subsequently introduced.

In one embodiment of the invention, the subject can be one in which suppression of an immune response is desired.

In one embodiment, the subject is a human affected by an inflammatory condition or disease/disorder, such as any of the diseases/disorders including, but not limited to, non-autoimmune inflammatory bowel disease, post-surgical adhesions, coronary artery disease, hepatic fibrosis, acute respiratory distress syndrome, acute inflammatory pancreatitis, endoscopic retrograde cholangiopancreatography-induced pancreatitis, burns, atherogenesis of coronary, cerebral and peripheral arteries, appendicitis, cholecystitis, diverticulitis, visceral fibrotic disorders, wound healing, skin scarring disorders (keloids, hidradenitis suppurativa), granulomatous disorders (sarcoidosis, primary biliary cirrhosis), asthma, pyoderma gandrenosum, Sweet's syndrome, Behcet's disease, primary sclerosing cholangitis, and an abscess.

In another embodiment, the subject is a human affected by an autoimmune condition or disease/disorder including, but not limited to, lupus erythematosus, pemphigus vulgaris, thyreoiditis, thrombocytopenic purpura, Graves disease, diabetes mellitus, juvenile diabetes, spontaneous autoimmune diabetes, myasthenia gravis, Addison's disease, an arthritic condition such as rheumatoid arthritis, ankylosing spondylitis, juvenile idiopathic arthritis, psoriatric arthritis; multiple sclerosis, psoriasis, uveitis, autoimmune hemolytic anemia, scleroderma, an intestinal inflammatory condition such as autoimmune inflammatory bowel disease, Crohn's disease, colitis, intestinal inflammation linked to food allergy; Sjorgen's disease, Hashimoto's disease, myasathenia gravis, Autoimmune Polyendocrinopathy syndromes, Type I diabetes mellitus (TIDM), autoimmune gastritis, autoimmune uveoretinitis, polymyositis, and thyroiditis, as well as in the generalized autoimmune diseases typified by human Lupus. "Autoantigen" as used herein refers to an antigen or epitope which is native to the mammal and which is immunogenic in said mammal disease.

The cells of the invention can also be used to prevent or treat transplantation reactions such as graft versus host disease (GVHD) and graft rejections.

Introduction of IL-13 producing Tr1-like cells into patients is performed using methods well known in the art such as adoptive cell transfer. Briefly, a mixed population of cells is extracted from a target donor. Depending on the application, the cells may be extracted during a period of remission, or during active disease. Typically this is done by collecting peripheral blood and harvesting white blood cells by leukapheresis (leukopheresis). For example, large volume leukapherisis (LVL) has been shown to maximize blood leukocyte yield. The harvested lymphocytes may be separated using the cell separation techniques based on Tr1-specific cell markers such as those described herein, and then transfused to a patient, typically the cell donor (except in GVHD where the donor and recipient are different), for adoptive immune suppression. Approximately 10³ to 10¹¹, preferably 10⁴ to 10¹⁰, preferably 10⁵ to 10⁹, preferably 10⁶ to 10⁹, more preferably 10⁶ to 10⁸ Tr1 cells are injected into the patient.

As used herein, the term "effective amount" means the amount of a therapeutic substance or composition which is sufficient to reduce (to any extent) or ameliorate the severity and/or duration of a disorder or one or more symptoms thereof, prevent the advancement of a disorder, cause regression of a disorder, prevent the recurrence, development, onset or progression of one or more symptoms associated with a disorder, detect a disorder, or enhance or improve the prophylactic or therapeutic effect(s) of another therapy (e.g., prophylactic or therapeutic agent). The effective amount will vary with the age, gender, race, species, general condition, etc., of the subject, the severity of the condition being treated, the particular agent administered, the duration of the treatment, the nature of any concurrent treatment, the pharmaceutically acceptable carrier used, and like factors within the knowledge and expertise of those skilled in the art. As appropriate, an "effective amount" in any individual case can be determined by one of ordinary skill in the art by reference to the pertinent texts and literature and/or by using routine experimentation, (for example, see Gennaro et al., Eds. Remington's The Science and Practice of Pharmacy, 20th edition, (2000), Lippincott Williams and Wilkins, Baltimore MD; Braunwald et al., Eds. Harrison's Principles of Internal Medicine, 15th edition, (2001), McGraw Hill, NY; Berkow et al., Eds. The Merck Manual of Diagnosis and Therapy, (1992), Merck Research Laboratories, Rahway NJ).

The terms "prevent," "preventing," and "prevention" refer herein to the inhibition of the development or onset of a disorder or the prevention of the recurrence, onset, or development of one or more symptoms of a disorder in a subject resulting from the administration of a therapy (e.g., a prophylactic or therapeutic agent), or the administration of a combination of therapies (e.g., a combination of prophylactic or therapeutic agents). By "treatment" or "treating" is meant that at least an amelioration of the symptoms associated with the disorder in the host is achieved, where amelioration is used in a broad sense to refer to at least a reduction in the magnitude of a parameter, e.g. symptom, associated with the condition being treated. As such, treatment also includes situations where the pathological condition, or at least symptoms associated therewith, are completely inhibited, e.g. prevented from happening, or stopped, e.g. terminated, such that the subject no longer suffers from the condition, or at least the symptoms that characterize the condition.

As used herein, the term "subject" refers to an animal, preferably a mammal and most preferably a human.

Another object of the invention is a method for increasing immune response of a transplanted cell population in a subject in need thereof, comprising administering to said subject an effective amount of a cell population depleted of IL-13 producing Tr1-like cells as described here above.

In one embodiment, said method is for treating a subject undergoing cancer treatment.

In one embodiment, the transplanted cell population is an antigen-specific effector T cell population. Preferably, said T cell population is specific for a tumor antigen such as MART-1 (Melan A) of melanoma or MAGE 1, 2, 3 of melanoma, thyroid medullary, small cell lung cancer, colon and/or bronchial squamous cell cancer...

In another embodiment, said method is for treating infectious diseases such as EBV, HIV, HCV, CMV infections.

Depletion of regulatory T cells has been shown to enhance vaccine-mediated immunity in cancer patients (Dannull et al., 2005, 115(12):3623-3633). Thus, in another embodiment of the invention, it is proposed that depletion of IL-13 producing Tr1-like cells may be used in cell therapy for treating cancer or infectious disease wherein said IL-13 producing Tr1-like cells may be deleterious or harmful for the treatment. For example, in cancer treatment, a transient depletion of IL-13 producing Tr1-like cells could be interesting before a vaccination/immunotherapy protocol, said transient depletion being obtained by depleting the blood of a patient ex vivo through a device according to the depletion method of the invention and re-injecting it immediately to the patient.

### BRIEF DESCRIPTION OF THE DRAWINGS

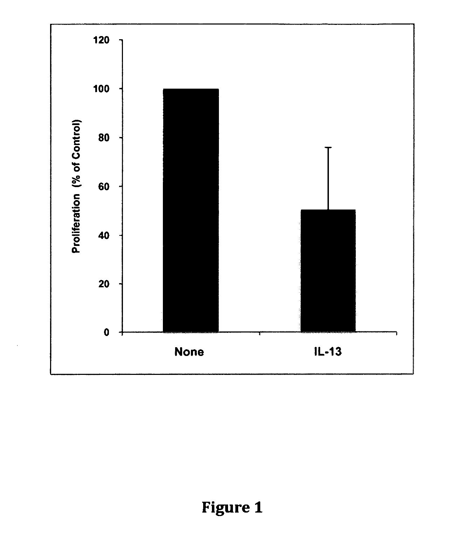

**Figure 1****: In vitro Inhibition of T-cell proliferative activity by IL-13.** Recombinant human IL-13 (12.5 ng/ml) was added to a culture of human PBMCs activated with anti-CD3 and anti-CD28 monoclonal antibodies. The proliferation of the cells was evaluated after 3 days of culture using viable cell labeling with WST-1.

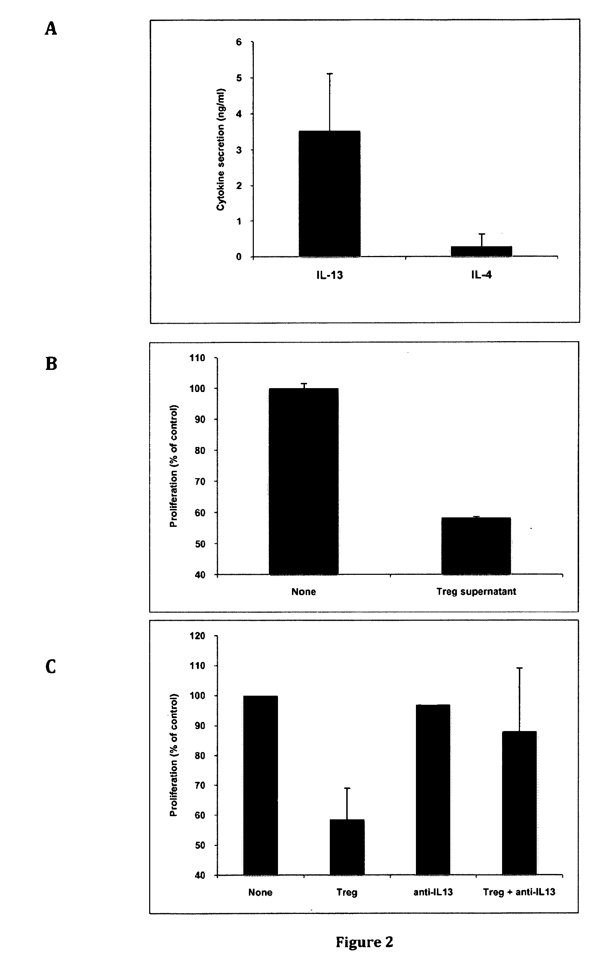

**Figure 2****: In vitro Inhibition of T-cell proliferative activity by IL-13 producing Tr1-like cells.** Panel A shows in vitro the cytokine secretion pattern of IL-13 producing regulatory cells after anti-CD3 + anti-CD28 monoclonal antibody treatment during 48hours. Panel B shows the suppressive potential of the IL-13 activated cell supernatant on the proliferation of human PBMC stimulated 3 days in culture with anti-CD3 + anti-CD28 monoclonal antibodies. Panel C, shows that addition of anti-IL13 blocking antibodies, is able to reverse the suppressive action of the supernatant of IL-13 producing regulatory cells.

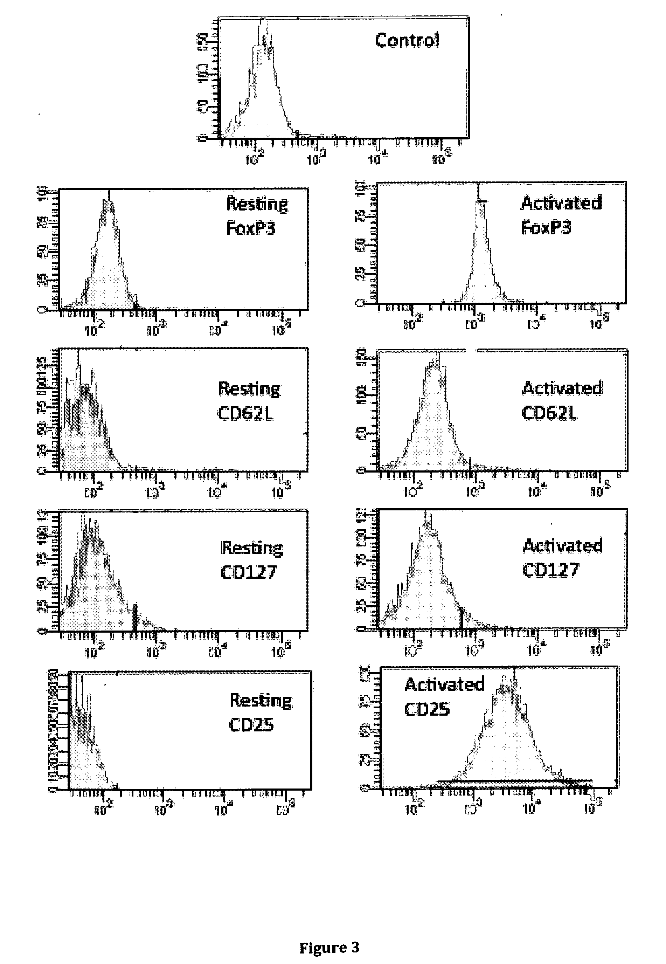

**Figure 3****: Phenotype of human IL-13 producing regulatory cells.** Human IL-13 regulatory cells were stained with fluorescent labeled CD25, CD127, CD62L or FoxP3 specific monoclonal antibodies for flow cytometry analysis. Cells were analyzed at a resting state or after 7 days of activation using IL-2 and CD3 + CD28 stimulating agents.

### EXAMPLES

### EXPERIMENTAL PROCEDURES

### IL-13 producing Tr1- like cell isolation and supernatant production

For IL-13 producing Tr1-like cell isolation, peripheral blood cells were separated by Ficoll density centrifugation and cultured at 2x10⁶ cells per ml in the presence of a specific antigen in order to allow the proliferation of antigen-specific Tr1-like cells. After 7 days of culture, cells were cloned by limiting dilution method during 3 weeks. Growing clones were then expanded using CD3/CD28 stimulatory agents and cytokines (IL-2 and IL-4). The Tr1-like cell identity of the clones was assessed by evaluating the phenotype by flow cytometry showing constitutive concomitant absence of CD62L and CD127 with expression of CD25 and Foxp3 only at the activated state. Production of Tr1-like cell supernatant was obtained by stimulating the cells with anti-CD3 + anti-CD28 coated magnetic beads (Dynal) during 2 days in X-vivo medium at 37°C +5%CO2.

### Flow cytometry studies

For flow cytometry studies on human cells, the following antibodies were used: PE-conjugated anti-CD127 (clone R34.34 from Beckman Coulter), FITC-labeled anti-CD4 Ab from Becton Dickinson (clone #RP4-T4), PeCy5-labeled anti-CD62L (clone Dreg56 from Becton Dickinson) and allophycocyanin-conjugated anti-CD25 (clone M-A251 from Becton Dickinson). Intracytoplasmic staining for human Foxp3 was performed using the PE-conjugated anti-Foxp3 Ab (clone 259 D/C7 from BD) and the intracytoplasmic staining kit (BD), according to the manufacturer's instructions.

### Cytokine assays

For the determination of the cytokine production profile, Tr1-like cell clones were stimulated with anti-CD3 + anti-CD28 monoclonal antibodies and the supernatants were harvested after 48 hours. ELISAs were performed using commercially available kits from Diaclone IFN-gamma and IL-4) and from e-Bioscience (IL-13). The experiments were performed following the manufacturer's instructions.

### Suppression studies

For suppression studies, Peripheral blood mononuclear cells were activated during two days with soluble anti-CD3 monoclonal antibody (1 microg/ml) in the presence or absence of IL-13 producing Tr1-like cell supernatant, anti-IL13 (clone 31606, R&D systems) or recombinant human IL-13 (6ng/ml, R&D systems). Proliferation of the incubated cells was measured using the WST1 Kit from Roche that allows evaluating the number of viable cells per culture well.

### RESULTS

### In vitro Inhibition of T-cell proliferative activity by IL-13

First, we wanted to demonstrate that recombinant human IL-13 is able to down regulate T cell proliferative activity in vitro. For this purpose, IL-13 was added to a culture of PBMCs activated with anti-CD3 and anti-CD28 monoclonal antibodies and the proliferation of the cells was measured after 3 days of culture (Figure 1). Results demonstrate that IL-13 is able to inhibit T- cell proliferation in vitro showing the immunosuppressive potential of this cytokine.

### In vitro Inhibition of T-cell proliferative activity by IL-13 producing Tr1-like cells

We thus wanted to know if IL-13 producing Tr1-like cells are able to inhibit bystander T-lymphocyte activity in the same kind of experimental settings using IL-13 as a suppressive cytokine. Figure 2A shows the cytokine secretion pattern of IL-13 regulatory cells activated by anti-CD3 and anti-CD28 monoclonal antibodies with high IL-13 but no IL-4 secretion. Figure 2B shows that supernatant of activated IL-13 producing regulatory cells are able to inhibit bystander T cell activation in culture. Importantly, this supernatant mediated suppression of T- cell proliferation was inhibited using IL-13 blocking antibodies (Figure 2C) showing that these cytokine represent a major suppressive soluble factor secreted by these cells.

### Phenotype of human IL-13 producing regulatory cells

Finally, we tested the expression of several regulatory cell markers by IL-13 regulatory cells and showed that the cells do not express the CD127, CD62L and the FoxP3 molecule. Moreover from the three markers, FoxP3 and CD25 are up regulated after activation (Figure 3).

## Claims

1. An isolated Tr1-like cell population capable of producing IL-13.

2. The isolated Tr1-like cell population according to claim **1**, wherein said cells produce low amounts of IL-4.

3. The isolated Tr1-like cell population according to claim **1** or **2**, wherein said cells express:
- CD4 and
- a low level of CD127 and
- a low level CD62L.

4. The isolated Tr1-like cell population according to anyone of claims **1** to **3**, wherein said cells are resting cells and express a low level of CD25 and a low level of FoxP3.

5. The isolated Tr1-like cell population according to anyone of claims **1** to **3**, wherein said cells are activated and express CD25 and an intermediate level of FoxP3.

6. An isolated Tr1-like clone capable of producing IL-13.

7. The isolated Tr1-like clone according to claim **6**, wherein said clone produce low amounts of IL-4.

8. The Tr1-like clone according to claim **7**, wherein said clone expresses:
- CD4 and
- a low level of CD127 and
- a low level CD62L.

9. A method of identifying an IL-13 producing Tr1-like cell population, comprising:
- detecting the cell surface expression of CD4, and at least one of CD127 and CD62L markers on a cell population,
- detecting the production of IL-13,
wherein the cells expressing CD4 and a low level of CD127 and/or a low level CD62L and producing IL-13, are the IL-13 producing Tr1-like cell population.

10. A method for enriching a cell population in IL-13 producing Tr1-like cells, comprising:
- identifying the cells expressing CD4 and a low level of CD127 and/or a low level CD62L and producing IL-13,
- selecting said cells,
thereby obtaining a cell population enriched in IL-13 producing Tr1-like cells wherein the percentage of IL-13 producing Tr1-like cells is at least twice the percentage of IL-13 producing Tr1-like cells before enrichment.

11. An enriched IL-13 producing Tr1-like cell population obtained according to claim **10**.

12. A kit for identifying or isolating an IL-13 producing Tr1-like cell population, comprising means for detecting the cell surface expression of CD4, CD25, CD127 and CD62L and means for detecting IL-13 production.

13. The isolated and/or enriched IL-13 producing Tr1-like cell population according to anyone of claims **1** to **5** or **11**, or the IL-13 producing Tr1-like clone according to anyone of claims **6** to **8** for preventing or treating an immune response, graft versus host disease and organ rejection, allergic disease, an inflammatory condition or an autoimmune condition.

14. A method for depleting a cell population in IL-13 producing Tr1-like cells, comprising:
- identifying the IL-13 producing Tr1-like cells according to claim 9,
- depleting said cells,
thereby obtaining a cell population depleted in IL-13 producing Tr1-like cells, wherein the percentage of IL-13 producing Tr1-like cells is at least 0.5 fold the percentage of IL-13 producing Tr1-like cells before depletion.

15. An IL-13 producing Tr1-like cells-depleted cell population obtained according to claim **14**.

16. The IL-13 producing Tr1-like cells-depleted cell population according to claim **15** for increasing an immune response in a subject in need thereof.
